**Europäisches Patentamt**

**European Patent Office**

**Office européen des brevets**

(11) Veröffentlichungsnummer : **0 141 922**
**B1**

(12) # EUROPÄISCHE PATENTSCHRIFT

(45) Veröffentlichungstag der Patentschrift :
28.09.88

(21) Anmeldenummer : 84109379.2

(22) Anmeldetag : 08.08.84

(51) Int. Cl.⁴ : **G 01 N 33/96,** G 01 N 33/92

(54) **Verfahren zur Verringerung einer Trübung in Kontrollseren.**

(30) Priorität : 19.08.83 DE 3329952

(43) Veröffentlichungstag der Anmeldung :
22.05.85 Patentblatt 85/21

(45) Bekanntmachung des Hinweises auf die Patenterteilung : 28.09.88 Patentblatt 88/39

(84) Benannte Vertragsstaaten :
AT BE CH DE FR GB IT LI LU NL SE

(56) Entgegenhaltungen :
DE-A- 2 825 391
DE-A- 3 017 152
DE-A- 3 107 060 .

(73) Patentinhaber : BEHRINGWERKE AKTIENGESELLS-
CHAFT
Postfach 1140
D-3550 Marburg/Lahn (DE)

(72) Erfinder : Steuer, Dagmar
Am Sonnenhang 9
D-3550 Marburg-Marbach (DE)
Erfinder : Schmidtberger, Rudolf
Salegrund 1
D-3550 Marburg 1 (DE)

(74) Vertreter : Meyer-Dulheuer, Karl-Hermann, Dr. et al
HOECHST Aktiengesellschaft Zentrale Patentabteilung Postfach 80 03 20
D-6230 Frankfurt/Main 80 (DE)

**Beschreibung**

Die Erfindung betrifft ein Verfahren zur Verminderung einer Trübung in einem getrockeneten und wieder aufgelösten Kontrollserum sowie ein getrocknetes Kontrollserum mit verminderter Trübung nach Wiederauflösen. Das Verfahren ist auch für Kontrollseren für Lipidbestimmungen geeignet, besonders solche mit erhöhtem Lipidgehalt.

Unter Kontrollseren sind Seren humanen oder tierischen Ursprungs mit gegebenenfalls veränderter, jedoch serumähnlicher Zusammensetzung zu verstehen, die Serumbestandteile in bekannter Konzentration enthalten und zur Kontrolle von Bestimmungsmethoden für diese Serumbestandteile geeignet sind.

Verfahren zur Herstellung derartiger Kontrollseren einschließlich der Einstellung einzelner Bestandteile auf gewünschte Gehalte sind bekannt.

Um die Haltbarkeit labiler Komponenten wie beispielsweise Enzyme oder Lipoproteine zu gewährleisten, können Kontrollseren lyophilisiert und bei niederer Temperatur gelagert werden. Unerwünschte Nebeneffekte einer Lyophilisation sind Trübungen, die nach Rekonstitution der Kontrollseren durch Veränderung des Lösungsverhaltens vor allem der Lipoproteine auftreten. Diese Trübungen stören häufig bei spektrophotometrischen Methoden, so daß ein Probenleerwert zusätzlich erforderlich ist. Besondere Probleme bereitet die Trübung bei Messungen im Bereich von 340 nm, in dem vor allem solche Enzymaktivitätsbestimmungen durchgeführt werden, die auf einer NADH/NAD-Messung beruhen. Die an sich schon hohe Extinktion von NADH wird durch die Trübung noch erhöht, so daß häufig in einem Bereich gemessen werden muß, in dem präzise Messungen nicht möglich sind. Die Ergebnisse werden ungenauer und hängen stark von der Güte des Photometers ab.

Es sind bereits Verfahren zur Vermeidung von Trübungen bekannt. Das Shock-Freezing (DE-A-22 43 014) erfordert einen großen technologischen Aufwand.

Der Einsatz gereinigter Lipidfraktion (Literatur: Clin. Chem. 22, (1976), 456-490 und 1299-1305) verursacht hohe Rohstoffkosten und die Zugabe von Detergentien kann den jeweiligen Test stören. Ein Zusatz von Zuckern, Zuckeralkoholen oder Aminozuckern (DE-A-28 25 391, Research Disclosure Oct. 1977, Nr. 16, 229, Clinical Abstracts, Vol. 87, 1977, Nr. 196 807 g und Vol. 90, 1979, Nr. 511 11c) bedingt eine hohe Viskosität und stört Glucose-Bestimmungsmethoden. Ein Zusatz organischer, nicht zuckerartiger Substanzen wie Methanol, Alanin, Triäthylenglycol, Valin, Acetat, Lactat oder Natrium-2-hydroxymethylbutyrat (DE-A-31 07 060) kann im Falle von Alanin und Methylbutyrat Störungen von Enzymreaktionen zur Folge haben. Ein Methanolzusatz ist allgemein gesundheitsgefährdend; wenn Natriumacetat verwendet wird, ist das Kontrollserum nicht mehr als Universal-Kontrollserum für Elektrolytbestimmungen einsetzbar; ein Zusatz von Ammoniumverbindungen stört bei Harnstoff-Bestimmungen; andere Substanzen können generelle Teststörungen verursachen.

Die Erfindung hat sich daher die Aufgabe gestellt, ein Universal- und besonders ein Lipid-Kontrollserum mit verminderter Trübungsneigung nach Rekonstitution einer getrockneten Form herzustellen, das die beschriebenen Nachteile der bekannten Verfahren nicht besitzt.

Überraschenderweise wurde gefunden, daß sich trübungsärmere rekonstituierte Kontrollseren herstellen lassen, wenn dem Kontrollserum vor der Trocknung Prolin zugesetzt wird.

Weiterhin wurde gefunden, daß eine Trübung durch zugesetzte Lipide, insbesondere Triglyceride, durch Kombination der genannten Aminosäure mit Na-Desoxycholat weiter vermindert wird, insbesondere dann, wenn das Kontrollserum einen reduzierten Elektrolytgehalt aufweist (elektrolytarm ist). Na-Desoxycholat eignet sich für diesen Zweck besonders, weil es selbst keine störende Trübung verursacht.

Gegenstand der Erfindung ist daher ein Verfahren zur Verringerung einer Trübung in einem getrockneten und rekonstituierten Kontrollserum, dadurch gekennzeichnet, daß dem Kontrollserum Prolin zugesetzt wird.

Eine besondere Ausgestaltungsform der Erfindung besteht darin, einem Kontrollserum mit erhöhtem Lipidgehalt weiterhin Na-Desoxycholat zuzusetzen. Dies ist besonders vorteilhaft, wenn die Ionenstärke des Kontrollserums gering, wenn es also elektrolytarm ist.

Die wirksame Konzentration für die genannte Aminosäure liegt zwischen 5-100 g/l, wobei die benötigte Menge abhängig vom Lipidgehalt des Serums ist. Natrium-Desoxycholat wird in einer Konzentration von 0,5-5 g/l eingesetzt.

« Elektrolytarm » soll im Rahmen der vorliegenden Erfindung heißen, daß die Konzentration von Natrium 40 bis 80 mmol/l und die der Chloridionen 20 bis 70 mmol/l ist. Das erfindungsgemäße Verfahren eignet sich zur Herstellung von klinisch-chemischen Kontrollseren, das heißt von Präparaten, die zur Qualitätskontrolle klinisch interessanter Serumparameter wie Enzym-, Substrat-, Metabolit-, Hormon- oder Elektrolytgehalt verwendet werden sollen. Es eignet sich ebenfalls zur Herstellung einer speziellen Lipid-Kontrolle oder einem Lipid-Calibrator. Die Reduktion der Trübung wurde folgendermaßen gemessen: Die Extinktion des mit destilliertem Wasser rekonstituierten getrockneten Kontrollserums mit einem oder beiden erfindungsgemäßen Zusätzen wurde mit einem Linienspektralphotometer bei 546 nm in einer Küvette mit 1 cm Schichtdicke gemessen und verglichen mit der Extinktion desselben Kontrollserums ohne Zusatz.

Unter Rekonstitution versteht man die Lösung eines Trockengutes in der Menge Lösungsmittel, in der es vor der Trocknung enthalten war.

**0 141 922**

Die folgenden Beispiele erläutern die Erfindung.

Beispiel

Als Serumbasis des Kontrollserums wurde gepooltes Humanserum von gesunden Spendern bzw. Humanserum mit reduziertem NaCl-Gehalt verwendet. Zur Erhöhung der Triglyceridkonzentration wurde Eigelb-Extrakt zugesetzt. Cholesterin wurde als Rindercholesterinkonzentrat zugegeben. Danach wurde Prolin und gegebenenfalls Na-Desoxycholat in der angegebenen Konzentration zugesetzt, anschließend keimfrei filtriert, in Flaschen abgefüllt und lyophilisiert. Nach Rekonstitution mit destilliertem Wasser wurde die Trübung gemessen.

Die folgende Tabelle gibt die Ergebnisse der Extinktionsmessung bei 546 nm wieder.

(Siehe Tabelle Seite 4 f.)

| Ausgangsmaterial | Cholesterin (mmol/l) | Triglyceride (mmol/l) | Na-Desoxycholat. (g/l) | Prolin (g/l) | Ext.546 nm |
|---|---|---|---|---|---|
| Normales Serum * | 3,1 | 0,91 | 0 | 0 | 0,616 |
|  | 3,1 | 0,91 | 0 | 35 | 0,383 |
|  | 3,1 | 0,91 | 0 | 115 | 0,139 |
|  | 4,91 | 0,91 | 0 | 0 | 1,100 |
|  | 4,91 | 0,91 | 0 | 50 | 0,602 |
|  | 4,91 | 0,91 | 2 | 50 | 0,200 |
|  | 7,76* | 0,91 | 2 | 50 | 0,265 |
|  | 5,69* | 2,29* | 2 | 0 | 1,035 |
|  | 5,69* | 2,29* | 2 | 50 | 0,614 |
|  | 7,76* | 2,29* | 2 | 0 | 1,304 |
|  | 7,76* | 2,29* | 2 | 10 | 1,219 |
|  | 7,76* | 2,29* | 2 | 50 | 0,728 |
| Elektrolytarmes Serum* | 4,58 | 0,67 | 0 | 0 | 0,450 |
|  | 8,53* | 3,49* | 0 | 0 | über 3 |
|  | 8,53* | 3,49* | 2 | 50 | 0,6-0,8 |
|  | 11,4 * | 5,28* | 0 | 0 | über 3 |
|  | 11,4 * | 5,28* | 2 | 50 | 0,7-0,8 |

* Die so gekennzeichneten Konzentrationen wurden durch Zugabe von Cholesterin oder Triglyceriden erhalten.

**0 141 922**

## Patentansprüche

1. Verfahren zur Verringerung einer Trübung in einem getrockneten und rekonstituierten Kontrollserum, dadurch gekennzeichnet, daß dem Kontrollserum vor dem Trocknen Prolin und gegebenenfalls Na-Desoxycholat zugesetzt wird.

2. Verfahren nach Anspruch 1, dadurch gekennzeichnet, daß im flüssigen Kontrollserum die Konzentration von Natrium 40-80 mmol/l und die der Chloridionen 20-70 mmol/l ist.

3. Verfahren nach Anspruch 1 oder 2, dadurch gekennzeichnet, daß im flüssigen Kontrollserum die Konzentration des Prolins 5-100 g/l ist.

4. Verfahren nach Anspruch 2 oder 3, dadurch gekennzeichnet, daß im flüssigen Kontrollserum die Konzentration von Natrium-Desoxycholat 0,5-5 g/l ist.

5. Kontrollserum in trockener Form, dadurch gekennzeichnet, daß es Prolin und gegebenenfalls Na-Desoxycholat enthält.

## Claims

1. A process for reducing turbidity in a dried and reconstituted control serum, which comprises adding proline and, if appropriate, Na deoxycholate to the control serum before drying.

2. The process as claimed in claim 1, wherein the concentration of sodium in the liquid control serum is 40-80 mmol/liter and that of the chloride ions is 20-70 mmol/liter.

3. The process as claimed in either of claims 1 and 2, wherein the concentration of the proline in the liquid control serum is 5-100 g/liter.

4. The process as claimed in either of claims 2 and 3, wherein the concentration of sodium deoxycholate in the liquid control serum is 0.5-5 g/liter.

5. A control serum in dry form, which contains proline and, if appropriate, Na deoxycholate.

## Revendications

1. Procédé pour la diminution d'un trouble dans un sérum témoin séché et reconstitué, caractérisé en ce que l'on ajoute de la proline et, éventuellement, du désoxycholate de Na au sérum témoin, avant le séchage.

2. Procédé selon la revendication 1, caractérisé en ce que, dans le sérum témoin liquide, la concentration du sodium va de 40 à 80 mmoles/litre, et celle des ions chlorure va de 20 à 60 mmoles/litre.

3. Procédé selon la revendication 1 ou 2, caractérisé en ce que dans le sérum témoin liquide, la concentration de la proline va de 5 à 100 g/litre.

4. Procédé selon la revendication 2 ou 3, caractérisé en ce que, dans le sérum témoin liquide, la concentration du désoxycholate de sodium est de 0,5 à 5 g/litre.

5. Sérum témoin sous forme séchée, caractérisé en ce qu'il contient de la proline et éventuellement du désoxycholate de Na.